# EUROPEAN PATENT SPECIFICATION

(11) **EP 2 901 950 B1**
(45) Date of publication and mention of the grant of the patent: **04.10.2017**
(21) Application number: 14200061.1
(22) Date of filing: 23.12.2014
(51) Int. Cl.: A61B 17/3209, A61B 18/14, A61B 17/00

(54) **Blade tip profile for use in cutting of tissue**
Klingenspitzenprofil zur Verwendung beim Schneiden von Gewebe
Profil de pointe de lame destiné à être utilisé dans la coupe de tissu

(30) Priority: 30.01.2014 US 201461933525 P; 11.11.2014 US 201414538274
(43) Date of publication of application: 05.08.2015
(73) Proprietor: Covidien LP, Mansfield, MA 02048 (US)
(72) Inventor: Stamm, Stephen J., Wheat Ridge, Colorado 80033 (US); Brandt, Kim V., Loveland, Colorado 80537 (US)
(74) Representative: Soames, Candida Jane

(56) References cited:
- EP-A2- 1 325 721
- WO-A1-01/68290
- WO-A1-03/066277
- WO-A1-2006/021269
- DE-A1-102004 052 068
- US-A1- 2009 012 520

## Description

### BACKGROUND

In certain surgical procedures, a forceps instrument is used which relies on mechanical action between its jaws to grasp, clamp and constrict vessels or tissue. Electrosurgical forceps may utilize both mechanical clamping action and electrical energy to effect hemostasis by heating tissue and blood vessels to coagulate and/or cauterize tissue. Some surgical procedures include more than simply cauterizing tissue, and rely on the unique combination of clamping pressure, precise electrosurgical energy control and gap distance (i.e., distance between opposing jaw members when closed about tissue) to "seal" tissue, vessels and certain vascular bundles. Typically, once a vessel is sealed, the surgeon has to accurately cut the vessel along the newly formed tissue seal. Accordingly, many vessel sealing instruments have been designed that incorporate a knife or blade member which effectively cuts the tissue after forming a tissue seal.

In certain surgical procedures, there may be many repetitions of cauterizing and cutting of tissue. Thus, a surgical knife which is capable of maintaining sufficient sharpness may be valuable. However, due to certain design and manufacturing issues of prior surgical knives, the tip of the knife edge may yield prematurely, thereby rendering the cutting edge too dull to properly cut the tissue.

EP-A-1325721 describes a blade for corneal surgery. The blade has a cutting edge adapted to incise a corneal epithelium but not to incise a Bowman's membrane which is harder than the corneal epithelium. The cutting edge has a height W1 at the tip of 1 to 70 µm, preferably 1 to 50 µm, more preferably 2 to 20 µm. The cutting edge has a radius of curvature R1 of 0.5 to 35 µm, preferably 0.5 to 25 µm. The angle θ1 formed by the upper and lower blade surfaces adjacent to the cutting edge is from 10 to 70 degrees, preferably 10 to 50 degrees, 15 to 40 degrees, for example about 19 degrees.

### SUMMARY

Due to the design and manufacturing issues of prior surgical knives, there may be a need for a surgical knife with a robust cutting edge that provides sufficient knife sharpness over many repetitions.

In a first aspect, the present invention provides a knife for cutting tissue comprising: a cutting edge including a tip, wherein a radius of the tip is between 0.18 µm (0.000007 inches) and 2.5 µm (0.0001 inches); and the cutting edge having a web thickness profile defined by a web thickness that varies as a function of distance from the tip, wherein at a distance of 76 µm (0.003 inches) from the tip, the web thickness is between 30 µm (0.0012 inches) and 46 µm (0.0018 inches).

Suitably, the cutting edge has a constant cutting edge angle of between 22 degrees and 31 degrees from the cutting edge tip to a distance of 76 µm (0.003 inches) from the cutting edge tip. In some embodiments, the knife may have a length between 178 mm (7 inches) and 279 mm (11 inches) and may have a width between 3.18 mm (0.125 inches) and 12.7 mm (0.5 inches). In certain examples, the knife material has a Rockwell C hardness value between 47 and 53 within a distance from the cutting edge tip of 76 µm (0.003 inches). The knife may be made from stainless steel or titanium. Additionally, the knife material may be heat treated or coated from a vapor deposition process.

In a second aspect, the present invention provides a medical device comprising: a shaft with a distal end and a proximal end; a handle coupled with the proximal end of the shaft, the handle comprising one or more triggers; a knife according to the present invention housed within the shaft, the knife configured to deploy and retract from the distal end of the shaft in response to an actuation of at least one of the one or more triggers.

In some embodiments, the medical device includes a pair of jaws coupled with the distal end of the shaft. The pair of jaws may be configured to hinge open and closed in response to an actuation of at least one of the one or more triggers.

In a further aspect, the present invention provides a method for making a knife for cutting tissue, comprising: forming a radius of a tip of the knife between 0.18 µm (0.000007 inches) and 2.5 µm (0.0001 inches); and forming a cutting edge of the knife having a web thickness profile defined by a web thickness that varies as a function of distance from the tip, wherein at a distance of 76 µm (0.003 inches) from the tip, the web thickness is between 30 µm (0.0012 inches) and 46 µm (0.0018 inches).

Suitably, the step of forming a cutting edge comprises forming a cutting edge having a constant cutting edge angle of between 22 degrees and 31 degrees from the cutting edge tip to a distance of 76 µm (0.003 inches) from the cutting edge tip.

Suitably, the step of forming the cutting edge includes a grinding process or a stamping process. Suitably, the method comprises using an electro polishing process to form the radius of the tip between 0.18 µm (0.000007 inches) and 2.5 µm (0.0001 inches). Suitably, the method comprises removing a burr at the cutting edge tip (310) and forming the cutting edge tip radius by an electro-polishing process.

In other embodiments, the step of forming the cutting edge comprises utilizing a photolithography process to form the radius of the tip between 0.18 µm (0.000007 inches) and 2.5 µm (0.0001 inches) and to form the web thickness profile of the cutting edge.

### BRIEF DESCRIPTION OF THE DRAWING

A further understanding of the nature and advantages of embodiments of the present invention may be realized by reference to the following drawings. In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.
FIG. 1 is a side, perspective view of a forceps provided in accordance with the present disclosure.
FIG. 2 is a side view of a distal end of the forceps of FIG. 1 wherein jaw members of the forceps are disposed in a spaced-apart position.
FIG. 3A is a side view of a knife in accordance with the present disclosure.
FIG. 3B is a detail view of the distal end of the knife of FIG. 3A.
FIG. 4 is a schematic view of a cutting edge profile of a knife in accordance with certain embodiments of the present disclosure.
FIG. 5A is a schematic view of a cutting edge profile of a knife in accordance with certain embodiments of the present disclosure.
FIG. 5B is a schematic view of a cutting edge profile of a knife in accordance with certain embodiments of the present disclosure.
FIG. 5C is a schematic view of a cutting edge profile of a knife in accordance with certain embodiments of the present disclosure.

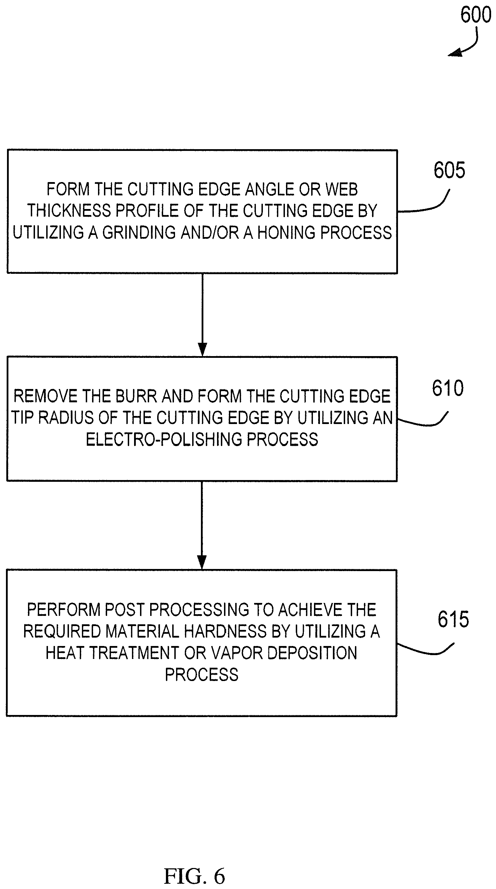

FIG. 6 is a flowchart of a method for forming a surgical knife in accordance with certain embodiments of the present disclosure.
FIG. 7 is a flowchart of a method for forming a surgical knife in accordance with certain embodiments of the present disclosure.
FIG. 8 is a flowchart of a method for forming a surgical knife in accordance with certain embodiments of the present disclosure.

### DETAILED DESCRIPTION

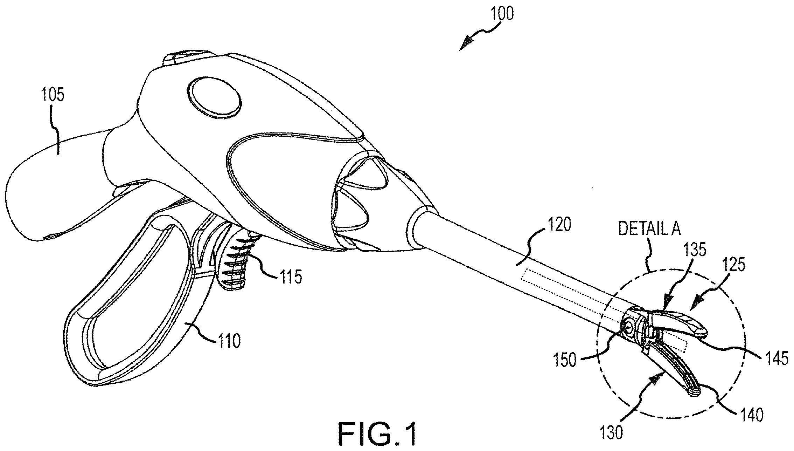

With reference to FIG. 1, an example of an electrosurgical forceps 100 device is shown. The forceps 100 are configured for use in various surgical procedures, and include a handle 105, a shaft 120, a first trigger 110, a second trigger 115, a jaw assembly 125, and a knife (not explicitly shown) that mutually cooperate to grasp, treat, and divide tubular vessels and vascular tissues. The jaw assembly 125 is coupled with the shaft 120 and includes jaws 130, 135. In some embodiments, one of jaws 130, 135 is configured to pivot about pivot pin 150 in response to an actuation of trigger 110, whereas the other jaw is configured to remain stationary. Such a configuration is termed unilateral pivoting. In some examples, configured for bilateral pivoting, each of jaws 130, 135 is configured to pivot about pivot pin 150 in response to an actuation of trigger 110.

A knife (not explicitly shown) is associated with the jaw assembly 125 and the shaft 120, and is configured to extend and retract from the distal end of shaft 120 in response to an actuation of trigger 115. The knife may have any length and width suitable for use in surgical procedures. In some embodiments, the knife may have a length of between 178 mm (7 inches) and 279 mm (11 inches) and may have a width of between 3.18 mm (0.125 inches) and 12.7 mm (0.5 inches). The distal end of the knife includes a cutting edge. The radius of the cutting edge tip ranges between 0.18 µm (0.000007 inches) and 2.5 µm (0.0001 inches), and at a distance of 76 µm (0.003 inches) from the tip, the web thickness ranges between 30 µm (0.0012 inches) and 46 µm (0.0018 inches). Each of the geometric parameters related to the knife and the cutting edge of the knife will be discussed in greater detail below.

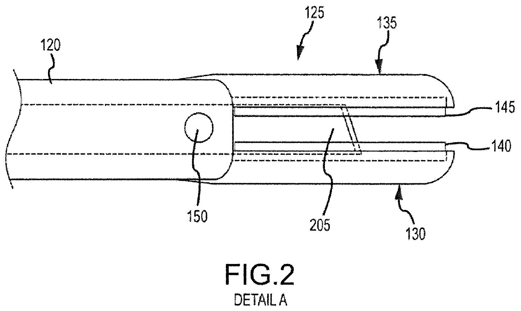

FIG. 2 is a detailed view of jaw assembly 125 of FIG. 1 showing the jaw assembly 125 in a closed position. Jaw assembly 125 may include an electrically conductive sealing plate 140, 145 which is electrically coupled with a power source (not shown) to deliver electrical energy, such as RF energy, to effect hemostasis by heating tissue and blood vessels to coagulate and/or cauterize tissue grasped between the jaws 130, 135. The power source may be external, or in some embodiments, may be internal such as a battery for example. FIG. 2 shows knife 205, which may be an example of the knife described in connection with FIG. 1 . The knife 205 may be associated with the shaft 120 and the jaws 130, 135 and may be configured to extend and retract from the distal end of shaft 120 in response to an actuation of trigger 115 to cut the cauterized tissue. In certain circumstances, the knife 205 may need to cut tissue grasped between jaws 130, 135 that has not been treated by RF energy. Untreated tissue is tougher than treated tissue and therefore requires a sharper and more robust cutting edge to accurately cut the tissue. According to various embodiments of the invention, a robust cutting edge of a knife may be defined by a combination of a cutting edge angle, a web thickness profile, and/or a radius of the cutting edge tip, as described in more detail in above and below.

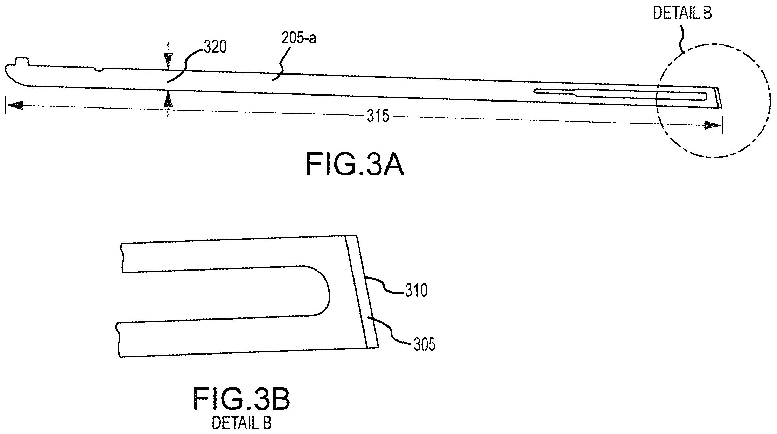

FIG. 3A shows an example of a knife 205-a according to various aspects of the invention, which may be an example of knife 205 from FIG. 1 or FIG. 2. In the illustrated example, the knife 205-a has a knife length 315, which in some examples may range between 178 mm (7 inches) and 279 mm (11 inches). In some embodiments, the knife length 315 may be approximately 234 mm (9.2 inches). Knife 205 also has a knife width 320, which in some examples may range between 3.18 mm (0.125 inches) and 12.7 mm (0.5 inches). In certain examples, the knife width 320 may be approximately 6.4 mm (0.25 inches). FIG 3B illustrates an example of a detailed view of the distal end of knife 205-a showing the cutting edge 305 and cutting edge tip 310 of knife 205-a. As will be discussed in more detail, the cutting edge 305 may be defined by a combination of a cutting edge angle and a radius of the cutting edge tip. In some embodiments, the cutting edge angle may range between 22 degrees and 31 degrees and the radius of the cutting edge tip may range between 0.18 µm (0.000007 inches) and 2.5 µm (0.0001 inches). The cutting edge 305 is defined by a combination of a web thickness profile and a radius of the cutting edge tip. The radius of the cutting edge tip ranges between 0.18 µm (0.000007 inches) and 2.5 µm (0.0001 inches), and at a distance of 76 µm (0.003 inches) from the tip, the web thickness ranges between 30 µm (0.0012 inches) and 46 µm (0.0018 inches).

Knife 205-a may be manufactured from various metallic materials such as stainless steel or titanium. However, it will be appreciated to those skilled in the art that other bio-compatible materials may also be used, such as silicone-based materials. In some embodiments, a material with a Rockwell C hardness value of greater than approximately 47 is used. In some examples, the Rockwell C hardness value of the knife material may range between 47 and 52. As will be discussed in more detail below, depending on the process used to fabricate the knife 205-a, the knife material may have a Rockwell C hardness value of between 47 and 53 either before or after the manufacturing process.

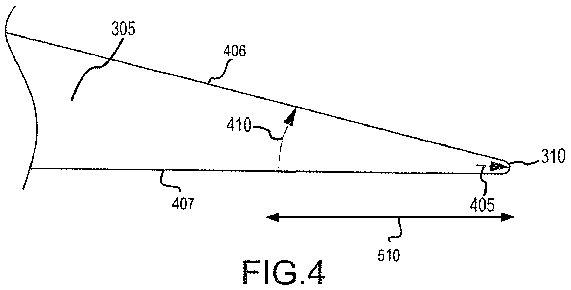

FIG. 4 shows a cross-sectional view illustrating an example of a cutting edge 305 of a knife. The cutting edge 305 may be an example of the cutting edge 305 of knife 205 from any of FIG.1, FIG.2, or FIG 3. The cutting edge 305 includes a cutting edge tip 310. The shape and size of the cutting edge tip may affect the sharpness and robustness of the cutting edge 305. In some embodiments of the invention, the tip 310 may be rounded and may be defined by a cutting edge tip radius 405. As described in detail below, the cutting edge tip radius 405 may be precisely measured and controlled through various manufacturing processes. Specifically, the cutting edge tip 310 is formed with a cutting edge tip radius 405 between 0.18 µm (0.000007 inches) and 2.5 µm (0.0001 inches). Controlling the shape and size of the cutting edge tip radius 405 may prevent premature dulling by removing cutting edge tip non-uniformities, such as a burr.

The cutting edge 305 may also include a cutting edge angle 410, which may be defined as the angle measured between the bottom surface 407 and top surface 406 of the cutting edge 305. The value of the cutting edge angle 410 may affect the sharpness and robustness of the cutting edge 305. For example, a cutting edge 305 with a lower cutting edge angle 410 may be sharper than a cutting edge 305 with a higher cutting edge angle 410. However, a lower cutting edge angle 410 may also result in a cutting edge 305 with less strength and robustness than a cutting edge 305 with a higher cutting edge angle 410. In some embodiments of the invention, the cutting edge angle may range between 22 degrees and 31 degrees.

A length measurement starting at the cutting edge tip 310 and extending parallel to the bottom surface 407 of cutting edge 305 is defined as a distance 510 from the cutting edge tip 310. In certain embodiments, the cutting edge angle 410 may be a constant value for all distances 510 from the cutting edge tip 310. Alternatively, the cutting edge 305 may include two or more portions, each with a different cutting edge angle 410. For example, within a distance 510 from the cutting edge tip 310 of approximately 76 µm (0.003 inches), the cutting edge angle 410 may be between 22 degrees and 31 degrees. However, for distances 510 from the cutting edge tip 310 of greater than approximately 76 µm (0.003 inches), the cutting edge angle 410 may be greater than 31 degrees or less than 22 degrees.

The values of the cutting edge angle 410 and the cutting edge tip radius 405 may be varied independently of one another to form various cutting edge profiles. As used herein, a cutting edge profile may refer to a particular combination of all the geometric parameters that define the cutting edge 305. Accordingly, a cutting edge profile of cutting edge 305 may be defined by the combination of a particular cutting edge tip radius 405 and a particular cutting edge angle 410. For example, a cutting edge profile may be defined by a cutting edge 305 with a cutting edge tip radius 405 equal to 2.5 µm (0.0001 inches) and a cutting edge angle 410 equal to 31 degrees. Another example of a cutting edge profile may be defined by a cutting edge 305 with a cutting edge tip radius 405 equal to 0.18 µm (0.000007 inches) and a cutting edge angle 410 equal to 22 degrees. By varying the cutting edge angle 410 and the cutting edge tip radius 405 within the ranges discussed above, a variety of cutting edge profiles may be defined that provide adequate sharpness to cut a wide variety of treated and/or untreated tissue while also providing sufficient strength to withstand yielding of the cutting edge.

The cutting edge profiles described herein are not limited to knives such as knife 205. Instead, the cutting edge profiles may be implemented with various types of surgical knives having a variety of shapes and sizes. Additionally, the cutting edge profiles may be used with knives that are compatible with various types of medical or surgical devices other than the electrosurgical forceps 100. Furthermore, the cutting edge profiles may be implemented with freestanding surgical knives that do not require a medical device assembly. Additionally, the cutting edge profiles may be used with knives with other applications other than surgery such as shaving, for example.

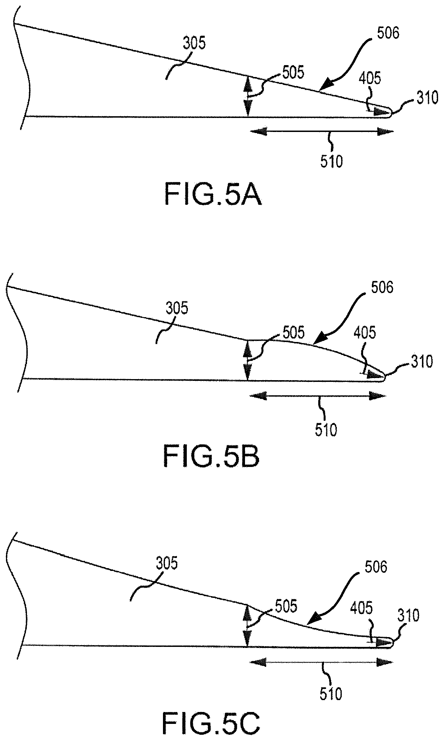

FIGS. 5A-5C show cross-sectional views illustrating examples of various embodiments of cutting edge 305 of a knife. Cutting edge 305 may be an example of the cutting edge 305 of knife 205 from any of FIG.1, FIG. 2, or FIG 3. A length measurement between the bottom surface 407 and the top surface 406 extending perpendicular to the bottom surface 407 may be defined as a web thickness 505. Furthermore, a web thickness profile 506 may be defined by the web thickness 505 as a function of the distance from the cutting edge tip 510. The web thickness profile may affect the sharpness and robustness of the cutting edge 305. According to the invention, at a distance 510 from the cutting edge tip 310 equal to 76 µm (0.003 inches), the web thickness 505 is between 30 µm (0.0012 inches) and 46 µm (0.0018 inches). From the cutting edge tip 310 up to a distance 510 from the cutting edge tip 310 of approximately 76 µm (0.003 inches), the shape of the web thickness profile 506 may vary. For example, referring to FIG. 5A, the web thickness profile 506 may be defined by a linear increase in web thickness 505 as the distance 510 from the cutting edge tip 310 increases. In some embodiments, the web thickness profile 506 may vary non-linearly as the distance 510 from the cutting edge tip 310 increases. For example, as shown in FIG. 5B, the web thickness profile 506 may be defined as a convex or generally outwardly-bulging shape. In yet another embodiment, as shown for example in FIG. 5C, the web thickness profile 506 may be defined as a concave or inwardly-curved shape.

According to the invention, a cutting edge profile is defined by a combination of a web thickness profile 506 and a cutting edge tip radius 405. As discussed above, the cutting edge tip radius 405 ranges between 0.18 µm (0.000007 inches) and 2.5 µm (0.0001 inches). In an embodiment of the invention, a cutting edge profile may be defined by a cutting edge tip radius 405 equal to 2.5 µm (0.0001 inches) and a web thickness profile 506 according to FIG. 5B , where at a distance 510 from the cutting edge tip 310 equal to 76 µm (0.003 inches), the web thickness is equal to 30 µm (0.0012 inches). By varying the web thickness profile 506 and the cutting edge tip radius 405 within the ranges discussed above, a variety of cutting edge profiles may be defined that provide adequate sharpness to cut a wide variety of treated and/or untreated tissue while also providing sufficient strength to withstand yielding of the cutting edge.

The novel cutting edge profiles described in connection with FIG. 1 to FIG. 5 may be formed using a variety of manufacturing techniques. In some examples, the entire cutting edge profile may be formed from a single manufacturing process. In other instances, the cutting edge profile may be formed by a combination of manufacturing processes. According to the invention, a manufacturing process may be utilized to form a web thickness profile 506 where at a distance 510 from the cutting edge tip 310 of 76 µm (0.003 inches), the web thickness 505 is between 30 µm (0.0012 inches) and 46 µm (0.0018 inches). Furthermore, the manufacturing process is utilized to form the cutting edge tip radius 405 between 0.18 µm (0.000007 inches) and 2.5 µm (0.0001 inches). The following are various examples of manufacturing processes for forming the cutting edge profiles within the numerical ranges listed above. For example, referring toFIG. 6, a manufacturing process 600 includes utilizing a grinding process 605 to grind the cutting edge 305 to form the cutting edge angle 410. Alternatively, the grinding process 605 may be utilized to form any of the web thickness profiles 506 described in connection with FIGS. 5A-5C. The grinding process 605 may be performed by a variety of grinding tools, such as an abrasive grinding wheel, for example. Examples of abrasive grinding wheels include silicon carbide, aluminum oxide, and/or diamond coated wheels.

Additionally, a honing process may be used either alone or in combination with the grinding process 605 to form either the cutting edge angle 410 or any of the web thickness profiles 506 shown in FIGS. 5A-5C . Typically, after the grinding process or honing process 605, a burr remains at the cutting edge tip 310. If this burr is not properly removed, the cutting edge tip 310 will yield and become dull prematurely during a surgical procedure. To remove this burr and to form the cutting edge tip radius 405, an electro-polishing process 610 may be utilized. By precisely controlling various control parameters of the electro-polishing process 610 such as process duration, temperature, voltage, and chemical composition, the cutting edge tip radius 405 may be formed and controlled. Due to the extremely small scale of the cutting edge tip radius 405, the radius can only be accurately measured using specialized equipment such as white light interferometry. Following the electro-polishing process 610, the knife material may be further processed 615 to achieve a Rockwell C hardness value of greater than approximately 47. In some embodiments, the post-manufacturing process 615 may be performed to achieve a Rockwell C hardness value of the knife material between 47 and 53. Examples of post-manufacturing processes include a heat treatment process or a vapor deposition process.

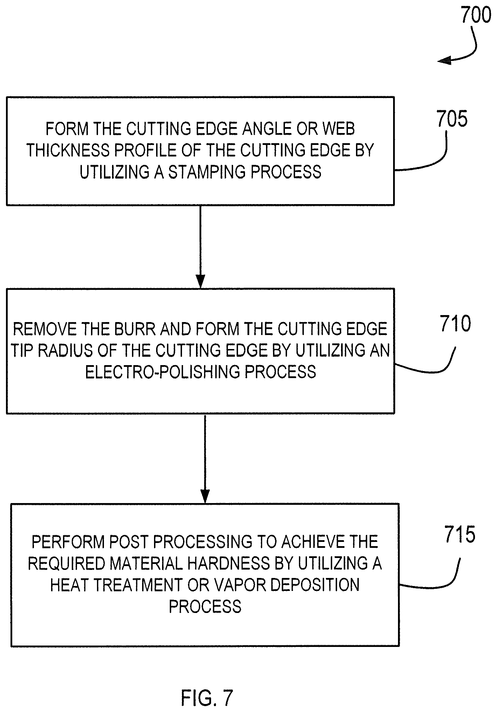

Referring to FIG. 7 , an example of a manufacturing process 700 is shown, which may be used to form cutting edge profiles according to various embodiments of the invention. Manufacturing process 700 includes a coining or stamping process 705 to form the cutting edge angle 410. Alternatively, the stamping process 705 may be utilized to form any of the web thickness profiles 506 shown in FIGS. 5A-5C. Similar to the grinding process 605 described above, typically, after the coining or stamping process 705, a burr remains at the cutting edge tip. To remove this burr and to form the cutting edge tip radius 405, an electro-polishing process 710 may be utilized. By precisely controlling various control parameters of the electro-polishing process such as process duration, temperature, voltage, and chemical composition, the cutting edge tip radius 405 may be formed and controlled. Following the electro-polishing process 710, the knife material may be further processed 715 to achieve a Rockwell C hardness value of greater than approximately 47. In some embodiments, the post-manufacturing process 715 may be performed to achieve a Rockwell C hardness value of the knife material between 47 and 53. Examples of post-manufacturing processes include a heat treatment process or a vapor deposition process.

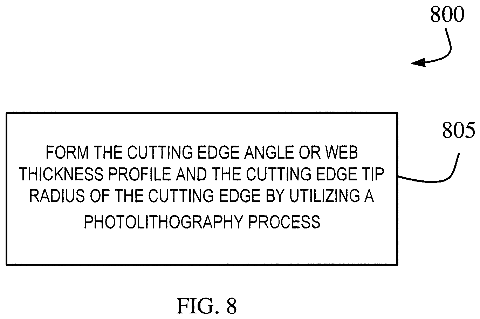

Referring to FIG. 8, a manufacturing process 800 may be used to form cutting edge profiles according to various embodiments of the invention. The manufacturing process 800 includes a photolithography process 805. One particular advantage of the photolithography process 805 is that the knife material may be hardened before the manufacturing process. The photolithography process 805 may used to form the cutting edge angle 410. Additionally, the photolithography process 805 may be used to form any of the web thickness profiles 506 shown in FIGS. 5A-5C. Another advantage of the photolithography process 805 may be that the process does not produce a burr at the cutting edge tip. Therefore, the photolithography process 805 may also be utilized to form the cutting edge tip radius 405 within the ranges previously discussed.

The foregoing descriptions of specific embodiments have been presented for purposes of illustration and description. They are not intended to be exhaustive or to limit the invention to the precise forms disclosed, and obviously many modifications and variations are possible within the scope of the accompanying claims.

## Claims

1. A knife (205, 205a) for cutting tissue comprising:
a cutting edge (305) including a tip (310), wherein a radius of the tip is between 0.18 µm (0.000007 inches) and 2.5 µm (0.0001 inches); and
the cutting edge having a web thickness profile (506) defined by a web thickness (505) that varies as a function of distance from the tip, wherein at a distance of 76 µm (0.003 inches) from the tip, the web thickness is between 30 µm (0.0012 inches) and 46 µm (0.0018 inches).

2. The knife of claim 1, wherein the cutting edge (305) has a constant cutting edge angle (410) of between 22 degrees and 31 degrees from the cutting edge tip (310) to a distance of 76 µm (0.003 inches) from the cutting edge tip.

3. The knife of any preceding claim, further comprising:
a knife length (315) between 178 mm (7 inches) and 279 mm (11 inches); and
a knife width (320) between 3.18 mm (0.125 inches) and 12.7 mm (0.5 inches).

4. The knife of any preceding claim, wherein within a distance of 76 µm (0.003 inches) from the tip (310), the knife comprises a material with a Rockwell C hardness between 47-53.

5. The knife of claim 4, wherein the material is stainless steel or titanium.

6. The knife of claim 4 or 5, wherein the material is coated from a vapor deposition process.

7. A medical device (100) comprising:
a shaft (120) with a distal end and a proximal end;
a handle (105) coupled with the proximal end of the shaft, the handle comprising one or more triggers (115);
a knife (205) according to any preceding claim housed within the shaft, the knife configured to deploy and retract from the distal end of the shaft in response to an actuation of at least one of the one or more triggers.

8. The medical device of claim 7, further comprising a pair of jaws (130, 135) coupled with the distal end of the shaft, wherein the pair of jaws is configured to hinge open and closed in response to an actuation of at least one of the one or more triggers (115).

9. A method for making a knife (205) for cutting tissue, comprising:
forming a radius of a tip (310) of the knife between 0.18 µm (0.000007 inches) and 2.5 µm (0.0001 inches); and
forming a cutting edge (305) of the knife having a web thickness profile (506) defined by a web thickness (505) that varies as a function of distance from the tip, wherein at a distance of 76 µm (0.003 inches) from the tip, the web thickness is between 30 µm (0.0012 inches) and 46 µm (0.0018 inches).

10. The method of claim 9, wherein the step of forming a cutting edge (305) comprises forming a cutting edge having a constant cutting edge angle (410) of between 22 degrees and 31 degrees from the cutting edge tip (310) to a distance of 76 µm (0.003 inches) from the cutting edge tip.

11. The method of claim 9 or 10, wherein forming the cutting edge (305) includes a grinding process or a stamping process.

12. The method of claim 9, 10 or 11, comprising using an electro polishing process to form the radius of the tip (310) between 0.18 µm (0.000007 inches) and 2.5 µm (0.0001 inches).

13. The method of any of claims 9 to 12, comprise removing a burr at the cutting edge tip (310) and forming the cutting edge tip radius by an electro-polishing process.

14. The method of claim 9, comprising utilizing a photolithography process to form the radius of the tip between 0.18 µm (0.000007 inches) and 2.5 µm (0.0001 inches) and to form the web thickness profile (506) of the cutting edge (305).

## Patentansprüche

1. Messer (205, 205a) zum Schneiden von Gewebe, umfassend:
eine Schneidkante (305), die eine Spitze (310) umfasst, wobei ein Radius der Spitze zwischen 0,18 µm (0,000007 Zoll) und 2,5 µm (0,0001 Zoll) liegt; und
wobei die Schneidkante ein Stegdickenprofil (506) aufweist, das durch eine Stegdicke (505) definiert ist und das sich als eine Funktion von einem Abstand von der Spitze verändert, wobei die Stegdicke in einem Abstand von 76 µm (0,003 Zoll) von der Spitze zwischen 30 µm (0,0012 Zoll) und 46 µm (0,0018 Zoll) liegt.

2. Messer nach Anspruch 1, wobei die Schneidkante (305) einen konstanten Schneidkantenwinkel (410) von zwischen 22 Grad und 31 Grad von der Schneidkantenspitze (310) bis zu einem Abstand von 76 µm (0,003 Zoll) von der Schneidkantenspitze aufweist.

3. Messer nach einem vorstehenden Anspruch, weiter umfassend:
eine Messerlänge (315) zwischen 178 mm (7 Zoll) und 279 mm (11 Zoll); und
eine Messerbreite (320) zwischen 3,18 mm (0,125 Zoll) und 12,7 mm (0,5 Zoll).

4. Messer nach einem vorstehenden Anspruch, wobei das Messer innerhalb eines Abstands von 76 µm (0,003 Zoll) von der Spitze (310) ein Material mit einer Rockwell-C-Härte zwischen 47-53 aufweist.

5. Messer nach Anspruch 4, wobei das Material Edelstahl oder Titan ist.

6. Messer nach Anspruch 4 oder 5, wobei das Material mittels eines Aufdampfungsprozesses beschichtet ist.

7. Medizinische Vorrichtung (100), umfassend:
einen Schaft (120) mit einem distalen Ende und einem proximalen Ende;
einen Handgriff (105), der mit dem proximalen Ende des Schaftes gekoppelt ist, wobei der Handgriff einen oder mehrere Auslöser (115) umfasst;
ein Messer (205) nach einem vorstehenden Anspruch, das innerhalb des Schaftes untergebracht ist, wobei das Messer dazu konfiguriert ist, um als Reaktion auf eine Betätigung von zumindest einem von dem einen oder den mehreren Auslösern von dem distalen Ende des Schaftes auszufahren und einzufahren.

8. Medizinische Vorrichtung nach Anspruch 7, weiter umfassend ein Paar von Klemmbacken (130, 135), die mit dem distalen Ende des Schaftes gekoppelt sind, wobei das Paar von Klemmbacken dazu konfiguriert ist, um als Reaktion auf eine Betätigung von zumindest einem von dem einen oder den mehreren Auslösern (115) auf- und zuzuklappen.

9. Verfahren zur Herstellung eines Messers (205) zum Schneiden von Gewebe, umfassend:
Formen eines Radius einer Spitze (310) des Messers zwischen 0,18 µm (0,000007 Zoll) und 2,5 µm (0,0001 Zoll); und
Formen einer Schneidkante (305) des Messers mit einem Stegdickenprofil (506), das durch eine Stegdicke (505) definiert ist und sich als eine Funktion von einem Abstand von der Spitze verändert, wobei die Stegdicke in einem Abstand von 76 µm (0,003 Zoll) von der Spitze zwischen 30 µm (0,0012 Zoll) und 46 µm (0,0018 Zoll) liegt.

10. Verfahren nach Anspruch 9, wobei der Schritt von einem Formen einer Schneidkante (305) umfasst Formen einer Schneidkante mit einem konstanten Schneidkantenwinkel (410) von zwischen 22 Grad und 31 Grad von der Schneidkantenspitze (310) bis zu einem Abstand von 76 µm (0,003 Zoll) von der Schneidkantenspitze.

11. Verfahren nach Anspruch 9 oder 10, wobei Formen der Schneidkante (305) einen Schleifprozess oder einen Stanzprozess umfasst.

12. Verfahren nach Anspruch 9, 10 oder 11, umfassend Verwenden eines Elektropoliturprozesses, um den Radius der Spitze (310) zwischen 0,18 µm (0,000007 Zoll) und 2,5 µm (0,0001 Zoll) zu formen.

13. Verfahren nach einem der Ansprüche 9 bis 12 umfassen Entfernen eines Grats an der Schneidkantenspitze (310) und Formen des Schneidkantenspitzenradius durch einen Elektropoliturprozess.

14. Verfahren nach Anspruch 9, umfassend Anwenden eines Photolithographieprozesses, um den Radius der Spitze zwischen 0,18 µm (0,000007 Zoll) und 2,5 µm (0,0001 Zoll) zu formen und um das Stegdickenprofil (506) der Schneidkante (305) zu formen.

## Revendications

1. Couteau (205, 205a) pour couper du tissu comprenant :
un bord de coupe (305) incluant une pointe (310), dans lequel un rayon de la pointe est entre 0,18 µm (0,000007 pouce) et 2,5 µm (0,0001 pouce) ; et
le bord de coupe ayant un profil d'épaisseur de l'âme (506) défini par une épaisseur de l'âme (505) qui varie comme une fonction de la distance depuis la pointe, dans lequel à une distance de 76 µm (0,003 pouce) de la pointe, l'épaisseur de l'âme est entre 30 µm (0,0012 pouce) et 46 µm (0,0018 pouce).

2. Couteau selon la revendication 1, dans lequel le bord de coupe (305) a un angle de bord de coupe constant (410) entre 22 degrés et 31 degrés depuis la pointe de bord de coupe (310) jusqu'à une distance de 76 µm (0,003 pouce) depuis la pointe de bord de coupe.

3. Couteau selon l'une quelconque des revendications précédentes, comprenant en outre :
une longueur de couteau (315) entre 178 mm (7 pouces) et 279 mm (11 pouces) ; et
une largeur de couteau (320) entre 3,18 mm (0,125 pouce) et 12,7 mm (0,5 pouce).

4. Couteau selon l'une quelconque des revendications précédentes, dans lequel à l'intérieur d'une distance de 76 µm (0,003 pouce) à partir de la pointe (310), le couteau comprend une matière avec une dureté Rockwell C entre 47 et 53.

5. Couteau selon la revendication 4, dans lequel la matière est de l'acier inoxydable ou du titane.

6. Couteau selon la revendication 4 ou 5, dans lequel la matière est revêtue par un processus de dépôt en phase vapeur.

7. Dispositif médical (100) comprenant :
un arbre (120) ayant une extrémité distale et une extrémité proximale ;
une poignée (105) couplée à l'extrémité proximale de l'arbre, la poignée comprenant une ou plusieurs gâchettes (115) ;
un couteau (205) selon l'une quelconque des revendications précédentes logé à l'intérieur de l'arbre, le couteau étant configuré pour se déployer et se rétracter à partir de l'extrémité distale de l'arbre en réponse à un actionnement d'au moins une de la ou des plusieurs gâchettes.

8. Dispositif médical selon la revendication 7, comprenant en outre un couple de mâchoires (130, 135) couplées à l'extrémité distale de l'arbre, dans lequel le couple de mâchoires est configuré pour s'articuler en position ouverte et fermée en réponse à un actionnement d'au moins une de la ou des plusieurs gâchettes (115).

9. Procédé de fabrication d'un couteau (205) pour couper du tissu, comprenant :
la formation d'un rayon d'une pointe (310) du couteau entre 0,18 µm (0,000007 pouce) et 2,5 µm (0,0001 pouce) ; et
la formation d'un bord de coupe (305) du couteau ayant un profil d'épaisseur de l'âme (506) défini par une épaisseur de l'âme (505) qui varie comme une fonction de la distance depuis la pointe, dans lequel à une distance de 76 µm (0,003 pouce) depuis la pointe, l'épaisseur de l'âme est entre 30 µm (0,0012 pouce) et 46 µm (0,0018 pouce).

10. Procédé selon la revendication 9, dans lequel l'étape de formation d'un bord de coupe (305) comprend la formation d'un bord de coupe ayant un angle de bord de coupe constant (410) entre 22 degrés et 31 degrés depuis la pointe de bord de coupe (310) jusqu'à une distance de 76 µm (0,003 pouce) depuis la pointe de bord de coupe.

11. Procédé selon la revendication 9 ou 10, dans lequel la formation du bord de coupe (305) inclut un processus de meulage ou un processus d'estampage.

12. Procédé selon la revendication 9, 10 ou 11, comprenant l'utilisation d'un processus de polissage électrolytique pour former le rayon de la pointe (310) entre 0,18 µm (0,000007 pouce) et 2,5 µm (0,0001 pouce).

13. Procédé selon l'une quelconque des revendications 9 à 12, comprenant l'enlèvement d'une bavure au niveau de la pointe de bord de coupe (310) et la formation du rayon de pointe de bord de coupe par un processus de polissage électrolytique.

14. Procédé selon la revendication 9, comprenant l'utilisation d'un processus de photogravure pour former le rayon de la pointe entre 0,18 µm (0,000007 pouce) et 2,5 µm (0,0001 pouce) et pour former le profil d'épaisseur de l'âme (506) du bord de coupe (305).
